# EUROPEAN PATENT APPLICATION

(11) **EP 4 324 908 A1**
(43) Date of publication of application: **21.02.2024**
(21) Application number: 22190802.3
(22) Date of filing: 17.08.2022
(51) Int. Cl.: C12M 1/42

(54) **END-EFFECTOR FOR CELL VOLUMETRIC STIMULATION**

(71) Applicant: International Centre For Genetic Engineering And Biotechnology - ICGEB, 34149 Trieste (IT); Università degli Studi di Siena, 53100 Siena (IT)
(72) Inventor: Prattichizzo, Domenico, 53100 Siena (IT); Zacchigna, Serena, 34149 Trieste (IT); Villani, Alberto, 53100 Siena (IT)
(74) Representative: Pace Napoleone, Maria

(57) **Abstract**

End-effector (1) for cell volumetric stimulation, the end-effector (1) comprising a stem element (2) having a first end (3) coupable to an air transmission system (4) for inserting air in, and expelling air from, the end-effector (1), a deformable membrane (5) connected to a second end (6) of the stem element (2), the deformable membrane (5) being configured for cell seeding, and an air chamber (6) formed between the second end (7) of the stem element (2) and the deformable membrane (5), wherein in an inflated configuration air is inserted into the air chamber (6) and in a deflated configuration air is expelled from the air chamber (6), thereby providing a pressure and volume variation of the deformable membrane (5), and wherein the stem element (2) is shaped as a deformable hollow cylinder.

## Description

The invention relates to an end-effector for the volumetric stimulation of cells and a corresponding system comprising a plurality of said end-effectors. Also, the invention relates to an apparatus comprising the end-effector or the corresponding system coupled to an actuation system as well as a method for manufacturing the end-effector. In addition, the invention relates to a method for cell volumetric stimulation using said end-effector.

Recent advancements in biological research have paved the way for investigation on correlation between mechanic stimulation and cell metabolic and genie expression. Last biological investigation outcomes have demonstrated the correlation between cellular processes, such as respiration, metabolic alterations or proliferation, and mechanical proprieties and physical solicitation of the living cells.

Mechanobiology is, therefore, that field of the biological sciences that proposes a systematic phenotypic and genie investigation on mechanics of the cells, studying the alteration of mechanics proprieties of the cells or their reaction at force and pressure stress. Cells are mechanically stimulated in a living environment as the human body that exert on them tensional, compressive, laminar, and twisting forces, moreover these stimuli may be directed along one, two or three axes and be constant, monotonous, periodic, or aperiodic in time.

However, reproducing the stress of a living environment in highly structured laboratory environment as the *in-vitro* or *ex-vivo* contexts is an open challenge. The *in-vitro* investigation is usually performed to reduce the observation uncertainties of a living complex organism as guinea pig, but vitro is also a static environment, without stress and mechanical stimuli.

The prior art systems for *in-vitro* mechanobiology investigation offer solutions for tensional and compressive mono and biaxial stimulation with different time profiles. However, the technologies currently available are not compatible with the standard laboratory glassware used for medium and high throughput biological and pharmaceutical screening.

Examples of the present disclosure seek to address or at least alleviate the above problems.

According to a first aspect of the invention there is provided an end-effector for cell volumetric stimulation, the end-effector comprising:
a stem element having a first end coupable to an air transmission system for inserting air in, and expelling air from, the end-effector;
a deformable membrane connected to a second end of the stem element, the deformable membrane being configured for cell seeding; and
an air chamber formed between the second end of the stem element and the deformable membrane, wherein in an inflated configuration air is inserted into the air chamber and in a deflated configuration air is expelled from the air chamber, thereby providing a pressure and volume variation of the deformable membrane, and
wherein the stem element is shaped as a deformable hollow cylinder.

In a second aspect of the invention there is provided a system for cell volumetric stimulation comprising:
a plurality of end-effectors according to the first aspect; and
at least a top plate configured to position each of the plurality of end-effectors in a corresponding well structure of a multi-well plate.

In a third aspect of the invention there is provided an apparatus for cell volumetric stimulation comprising:
at least an end-effector according to the first aspect or at least a system according to the second aspect; and
an actuation system having an air transmission system housed at least in part in the stem element of the end-effector for generating volumetrically constant, monotonous, or user defined rhythmic stimulation on cells, wherein in particular the actuation system comprises mechanical, electronic and/or hydraulic components for measuring and controlling physical parameters on the deformable membrane and a microcontroller connected to said mechanical, electronic and/or hydraulic components.

In a fourth aspect of the invention there is provided a method for manufacturing an end-effector for cell volumetric stimulation according to the first aspect, the method comprising:
forming a membrane layer and a stem layer by sequential pouring bi-component silicone rubber in a membrane mold and a stem mold, respectively,
interrupting the sequential pouring by the insertion of a tin sphere to form an internal cavity between the membrane layer and the stem layer, the internal cavity functioning as the air chamber.

In a fifth aspect of the invention there is provided a method for cell volumetric stimulation using the end-effector according to the first aspect, or the system according to the second aspect, or the apparatus according to the third aspect, the method comprising:
providing at least one end-effector;
seeding the cells on an external surface of the deformable membrane of said end-effector;
placing the end-effector at least partially in a growth medium so that at least the cells are completely immersed in said growth medium; and
inserting air into the air chamber and expelling air from the air chamber in a continuous cycle, thereby providing a pressure and volume variation of the deformable membrane and obtaining cells stimulation.

Other aspects and features are defined in the appended claims.

Examples of the disclosure may make it possible to carry out a volumetric stimulation of cells in an *in-vitro* context using an end-effector designed to be possibly incorporated with standard laboratory techniques and methods. This particular end-effector is a robotic highly deformable and inflatable device able to harbor and volumetric stimulate/stress cells *in-vitro.*

Examples of the disclosure will now be described by way of example only with reference to the accompanying drawings, in which like references refer to like parts, and in which:
- Figure 1: shows a schematic representation of the end-effector according to an example.
- Figure 2: shows functioning of the end-effector according to an example.
- Figures 3A-B: show flowcharts of the method for manufacturing the end-effector according to an example.
- Figure 4: shows a schematic representation of the manufacturing method steps.

- Figure 5: shows a schematic representation of the system for cell volumetric stimulation according to an example.
- Figure 6: shows a schematic representation of the apparatus for cell volumetric stimulation according to an example.
- Figure 7: shows a flowchart of the method for using the end-effector according to an example.

An end-effector, a system, an apparatus and method for volumetric cell stimulation as well as a manufacturing method is disclosed. In the following description, a number of specific details are presented in order to provide a thorough understanding of the examples of the disclosure. It will be apparent however to a person skilled in the art that these specific details need not be employed in order to practice the examples of the disclosure. Conversely, specific details known to the person skilled in the art are omitted for the purposes of clarity in presenting the examples.

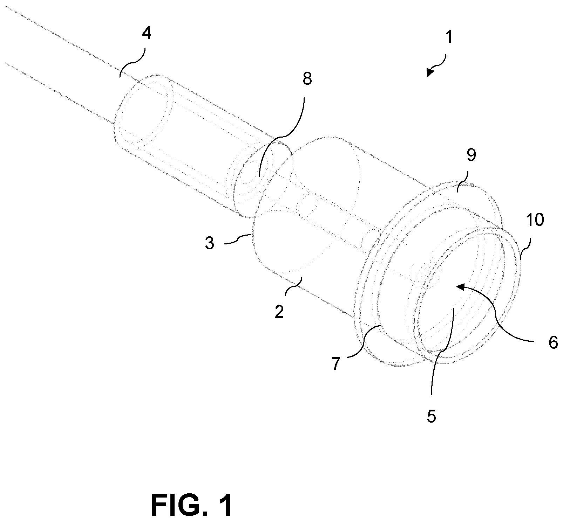

With reference to figure 1, the end-effector 1 used for the volumetric stimulation of cells comprises at least a stem element 2 a deformable membrane 5 and an air chamber 6 located between the stem element 2 and the membrane 5. The stem element 2 comprises a first end 3 and a second end 7 opposite to the first end 3. The first end 3 can be coupled to an air transmission system 4, for example through a pipe, so that air flow can be inserted in, or expelled from, the end-effector 1. The second end 7 of the stem element 2 is connected to the deformable membrane 5, the membrane 5 being deformed by air pressure due to the air flow variation in the end-effector 1. In particular, since the air chamber 6 is in contact with the membrane 5 (the membrane 5 form a portion of the air chamber 6), by inserting air into the air chamber 6 (inflated configuration), air is pushed on the membrane 5 and consequently the volume of the membrane is varied. The deformable membrane 5 is configured for cell seeding. Accordingly, a volume variation of the membrane 5 translates into a cell volumetric stimulation.

As mentioned, the air chamber 6 is formed in part by the deformable membrane 5 and in part by the internal walls of the stem element 2. For this reason, the stem element 2 is shaped as a deformable hollow cylinder. It is noted that the membrane 5 has a first side (internal side) forming a portion of the air chamber 6 or being in direct contact with the air chamber 6, and a second side (external side) opposite to the first side that is used as cell culture substrate. The second side expands in a growth medium 16 by deformation of the membrane 5.

In examples, the end-effector 1 comprises a needle structure 8 located at the first end 3 of the stem element 2 for air passage between inside and outside the air chamber 6. In other words, the air passes from the air transmission system 4 (e.g. a pipe) to the air chamber 6 for deforming the membrane 5 through the needle structure 8 that is firmly hold by the stem element 2.

In examples, the stem element 2 comprises a radial flap 9 at the second end 7. This flap 9 fosters the manipulation of the end-effector 1 by suitable holding tools such as a tweezer.

In examples, the deformable membrane 5 comprises an extruded confining rim 10. This rim 10 serves to better confine the cells that are seeded on the membrane 5.

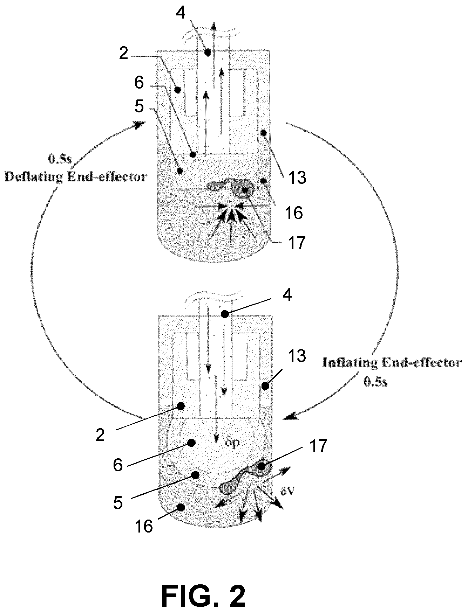

Figure 2 shows the application of the end-effector 1 for cell stimulation. The end-effector 1 has the shape of a small hollow cylinder that can be dimensionally integrated with standard laboratory tools, glassware, and technics, which are usually involved in mechanobiology research. The cells 17 are seeded on the deformable membrane 5 and once adhered, the end-effector 1 is immersed in growth medium 16 inside a well structure 13, for example of a cell culture plate. The top of figure 2 shows the deflated configuration when air is not present or is expelled from the air chamber 6. When the end-effector 1 is immersed in the growth medium 16, it finds itself in this deflated configuration. The bottom of figure 2 shows, on the other hand, the inflated configuration when air is inserted into the air chamber 6. The inflated configuration occurs when the end-effector 1 is already immersed in the grow medium 16. In this configuration, an air pressure variation (δp) is present in the air chamber 6 and the membrane 5 as well as the cells 17 will be subject to a volume variation (δv).

In examples, in the deflated configuration the surface of the deformable membrane 5 is flat and in the inflated configuration the surface of the deformable membrane is curved and is protruding outwards. Specifically, the flat surface fosters adhesion and equal redistribution of the cells 17 on the membrane 5 and the end-effector 1 is configured such that in the inflated configuration the curved protruding membrane 5 is always immersed in the growth medium 16 inside the well structure 13.

A continuous cycle of inflation and deflation is carried out to generates variations in pressure and volume of the membrane 5, thereby stressing the cells 17. As shown by the circular arrows in figure 2, the cyclic inflation and deflation process can occur each 0.5 seconds.

In examples, the stem element 2 and the deformable membrane 5 are both made of polymeric material, in particular of platinum catalyzed bi-component silicone rubber. Silicone rubber is biocompatible, hydrophobic, and can be sterilized by 70% ethanol or autoclaving.

In examples, the stem element 2 and the deformable membrane 5 are both made of transparent material. The transparency of the material fosters the cell imaging by fluorescence microscopes.

The membrane is designed to seed and harbor the cells on it. In examples, the deformable membrane 5 is made of an hyper-elastic material, in particular a Neo-Hookean material. In this way, by inflating air in the air chamber 6, the membrane 5 is mostly affected by the pressure variation. Also, the selected Neo-Hookean material guarantees the adhesion of the living cells and their survival.

In examples, the stem element 2 and/or the deformable membrane 5 are both made of biomaterials. In this way, no rejection problems are encountered during the cell culture in the growth medium 16.

In examples, the stem element 2 and/or the deformable membrane 5 are both made of hydrophobic materials. In this way, the end-effector can maintain its elastic properties even in a moist environment, such as an incubator.

In examples, the hardness of the material for realizing the stem element 2 is greater than the hardness of the material for realizing the deformable membrane 5, wherein in particular the material for realizing the stem element 2 has hardness higher than 40, in particular 50, on Shore scale A and the material for realizing the deformable membrane 5 has hardness lower than 40, in particular 30, on Shore scale 00.

In examples, the stem element 2 is made of a material layer having a thickness comprised between 7 mm and 9 mm, in particular of 8 mm, and the deformable membrane 5 is made of a material layer having a thickness comprised between 1 mm and 3 mm, in particular of 2 mm.

In examples, the stem element 2 has a main body having a width comprised between 5 mm and 10 mm, in particular 9 mm in order to be compatible with standard laboratory glassware for medium and high throughput screening (e.g. 24-wells and 96-wells plates).

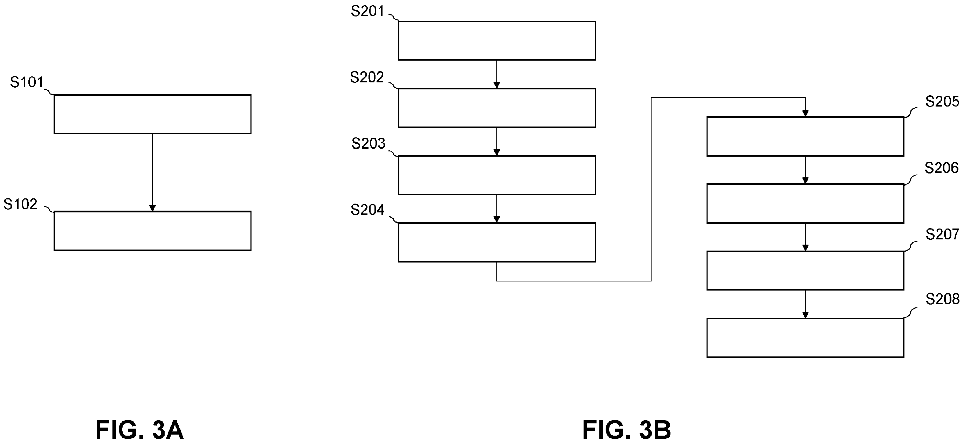

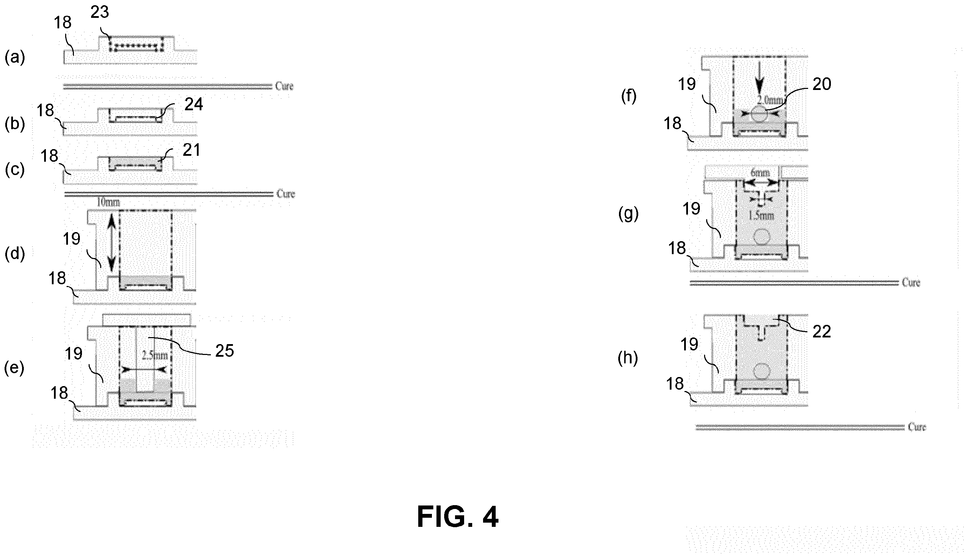

Figures 3A and 3B show two flow charts describing the steps of a method for manufacturing the end-effector 1. These steps are shown in a schematic representation in figure 4.

At step S101, a membrane layer 21 and a stem layer 22 are formed by sequential pouring bi-component silicone rubber in a membrane mold 18 and a stem mold 19, respectively. At step S102, the sequential pouring is interrupted by the insertion of a tin sphere 20 to form an internal cavity between the membrane layer 21 and the stem layer 22, wherein the internal cavity functions as the air chamber 6.

In examples, the method comprises:
coating a membrane mold 18 with resin, in particular polyepoxides, and curing for a first curing time, in particular 72 hours (at step S201);
coating the membrane mold 18 with released release agents 24 (at step S202);
pouring a first quantity of mixed bi-component silicone rubber, for example 1.27 g, in the membrane mold 18 and curing for a second curing time, in particular 3 hours, for example at 23°C, to form the membrane layer 21 (at step S203);
placing a guide 25 for the tin sphere, pouring a second quantity of mixed bi-component silicone rubber, for example 2.0 g, and curing for a third curing time, in particular 3 hours, for example at 23°C (at step S204);
removing the guide 25 and inserting the tin sphere 20 (at step S205);
pouring a third quantity of mixed bi-component silicone rubber, for example 3.1 g, and curing for a fourth curing time, in particular 3 hours, for example at 23°C (at step S206); placing a pinned plane 26 in the stem mold 19 to generate a needle guide (at step S207); and
pouring a silicone rubber harder than the mixed bi-component silicone rubber in the stem mold 19 and curing for a fifth curing time, in particular 12 hours, for example at 23°C, to form the stem layer 22 (at step S208).

In other words, each end-effector 1 consists of a soft hollow cylinder realized by layering transparent platinum catalyzed silicone rubber with hardness 30 on Shore scale 00 according to ASTM D2240 standard. Two main layers, the membrane 5 (the thinner, e.g. 2 mm) and the stem element 2 (the thicker, e.g. 8mm) are realized as sub-sequential pouring in Acrylonitrile butadiene styrene ((C8H8)x·(C4H6)y·(C3H3N)z) molds a bi-component silicone rubber, interrupted by the insertion of a tin sphere 20 to realize the internal cavity (e.g., radius 1 mm) designed to collect air and expand the membrane 5. The realization steps shown in figure 4 are tested to ensure the repeatability of mechanical proprieties of the effector batch. It is noted that the platinum catalyzer of the material of the membrane 5 and stem element 2 fosters the layering process and the cavitation by tin sphere 20. The electron exchange between platinum and tin induces the platinum crystallization and the detachment of the platinum from aluminum oxide support, and then, the inhibition of catalyzer in contact surface between tin sphere 20 and rubber.

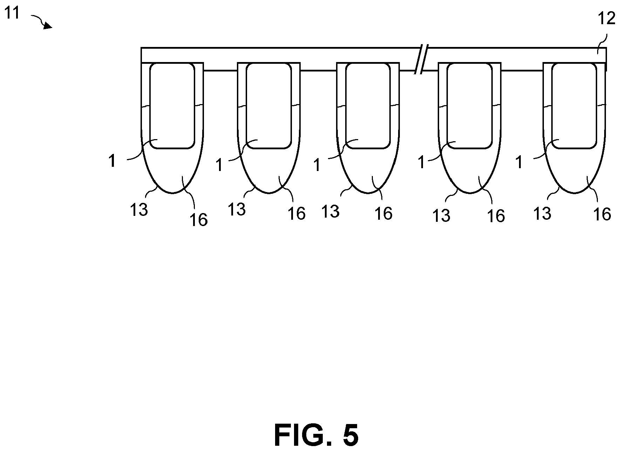

Figure 5 shows a cell volumetric stimulation system 11. The system 11 comprises a plurality of end-effectors 1. Also, the system comprises a top plate 12 configured to position each of the plurality of end-effectors 1 in a corresponding well structure 13 of a multi-well plate. The end-effectors 1 can be advantageously arranged along a line. Alternatively, the end-effectors 1 can be arranged in rows and columns so to be suitable for using in combination with standard cell culture plates provided with a plurality of wells 13 (arranged in rows and columns) filled with growth medium 16.

In examples, the top plate 12 is configured as a rigid lid, in particular of plastic, for ensuring both protection from contamination and recirculation of metabolic gasses and oxygen in a growth medium 16 present in the well structure 13. In particular, two different top plates 12 can be designed to place in same time up to 24 or 96 end-effectors 1 in the 24-wells and 96-wells plate, respectively. The top plates 12 are rigid plastic lids designed to route the end-effectors 1 into a multi-wells plate ensuring both protection from contamination by precipitation of suspended environmental corpuscles, and recirculation of metabolic gasses and oxygen avoiding cellular hypoxia.

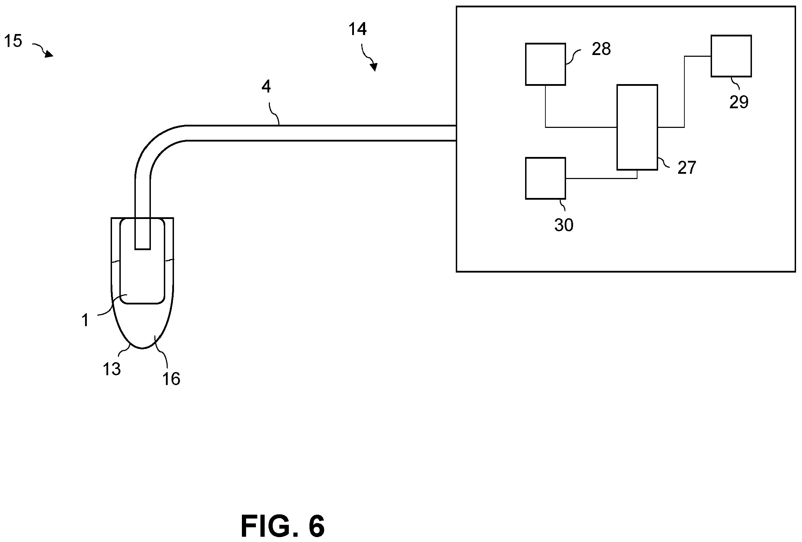

Figure 6 shows an apparatus 15 for volumetric stimulation of cells. The apparatus 15 comprises at least an end-effector 1 or at least a system 11 as described above coupled to an actuation system 14. In particular, the actuation system 14 is coupled to the apparatus through an air transmission system 4 that is housed at least in part in the stem element 2 of the end-effector 1. The actuation system 14 comprises components 28, 29, 30 for measuring and controlling physical parameters on the deformable membrane 5 and a microcontroller 27 connected to said components 28, 29, 30. For example, the components can be at least a pump 28, at least a sensor 29, in particular a pressure sensor, and at least a valve 30 or any electronics to generate volumetrically constant, monotonous, or user defined rhythmic stimulation on cells 17. The actuation system 14 can advantageously comprise a pneumatic actuator able to simultaneously inflate several end-effectors 1. The microcontroller 27 can regulate by valve the air flow routed in the pipes from the pumps 28 to the end-effectors 1, according with the measure of the pressure sensors 29, closing a loop control. The actuation system 14 is connected to the end-effector 1 by pipes 4 to delocalize the electronic component from incubator and to avoid the contamination of the cells 17 by oxides released from electronics and silicon due the humidity of the environment.

In examples, the apparatus 15 further comprises an analogical or digital I/O interface for controlling and setting deformation parameters of the cells.

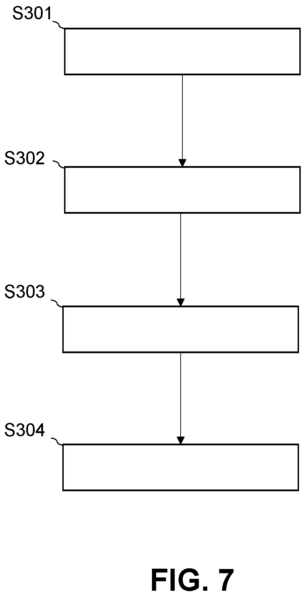

With reference to figure 7, the steps of a method for cell volumetric stimulation are shown. The method is carried out using the end-effector 1, the system 11, or the apparatus 15 as described above.

At step S301, at least one end-effector 1 is provided and at step S302 cells 17 are seeded on an external surface of the deformable membrane 5 of the end-effector 1. The end-effector 1 is placed at least partially in a growth medium 16 at step S303 so that at least the cells 17 are completely immersed in said growth medium 16. Specifically, the external side of the membrane 5 is completely immersed in the growth medium 16. At step S304, air is inserted into, and expelled from, the air chamber 6 in a continuous cycle. Accordingly a pressure and volume variation of the deformable membrane 5 is provided and cells stimulation is obtained.

Before seeding the cell 17 on end-effector membrane 5, three-step sterilization procedure and a subsequent coating treatment can be performed, thereby fostering the adhesion and survival of the cells 17 on the deformable membrane 5. The end-effectors sterilization procedure consists in a manual removal of macroscopic corpuscles by means of adhesive paper, followed by a soaking in 70% ethanol (CH3CH2OH) cleaning reagent for e.g. 900 sec. Then, to remove residual ethanol from the membrane 5, the end-effectors 1 are washed, for example with the Phosphate-Buffered Saline solution (PBS), a water-based salt solution commonly used in biological research. Then, the membrane 5 is coated with an adhesive glycoprotein, the fibronectin in solution with gelatin, a heterogeneous mixture of water-soluble proteins that are present in collagen. The coating procedure is aimed at ensuring the attachment of cells 17 on the membrane 5 and requires a waiting time of at least 2 hours to be efficacious.

The cells 17 are seeded on the membrane 5, placed in growth medium 16, and located inside a cell incubator to ensure the survival of the cells 17 for long period. The actuation system 11 provides the air flow from outside of incubator to the end-effectors 1 to blow them and stimulate the cells 17 inside the medium 16. The air pass from pipe 4 to end-effector 1 by the needle 8 inserted from the bottom part of the end-effector 1, the stem element 2, to the internal air chamber 6. Users can perform the settings of the device by analogical I/O interface on case of the actuation system 11 or through graphic interface on computer, choosing deformation trend and amplitude, frequency, or loading and selecting a deformation time series appropriately and in advance realized.

Although a variety of techniques and examples of such techniques have been described herein, these are provided by way of example only and many variations and modifications on such examples will be apparent to the skilled person and fall within the spirit and scope of the present invention, which is defined by the appended claims and their equivalents.

## Claims

1. End-effector (1) for cell volumetric stimulation, the end-effector (1) comprising:
a stem element (2) having a first end (3) coupable to an air transmission system (4) for inserting air in, and expelling air from, the end-effector (1);
a deformable membrane (5) connected to a second end (6) of the stem element (2), the deformable membrane (5) being configured for cell seeding; and
an air chamber (6) formed between the second end (7) of the stem element (2) and the deformable membrane (5), wherein in an inflated configuration air is inserted into the air chamber (6) and in a deflated configuration air is expelled from the air chamber (6), thereby providing a pressure and volume variation of the deformable membrane (5), and wherein the stem element (2) is shaped as a deformable hollow cylinder.

2. End-effector (1) according to claim 1, further comprising a needle structure (8) located at the first end (3) of the stem element (2) for air passage between inside and outside the air chamber (6).

3. End-effector (1) according to any one of claims 1 to 2, wherein
a. the stem element (2) and the deformable membrane (5) are both made of polymeric material, in particular of platinum catalyzed bi-component silicone rubber; and/or
b. the stem element (2) and the deformable membrane (5) are both made of transparent material; and/or
c. the deformable membrane (5) is made of an hyper-elastic material, in particular a Neo-Hookean material; and/or
d. the stem element (2) and/or the deformable membrane (5) are both made of biomaterials; and/or
e. the stem element (2) and/or the deformable membrane (5) are both made of hydrophobic materials.

4. End-effector (1) according to any one of claims 1 to 3, wherein the hardness of the material for realizing the stem element (2) is greater than the hardness of the material for realizing the deformable membrane (5), wherein in particular the material for realizing the stem element (2) has hardness higher than 40, in particular 50, on Shore scale A and the material for realizing the deformable membrane (5) has hardness lower than 40, in particular 30, on Shore scale 00.

5. End-effector (1) according to any one of claims 1 to 4, wherein the stem element (2) is made of a material layer having a thickness comprised between 7 mm and 9 mm, in particular of 8 mm, and the deformable membrane (5) is made of a material layer having a thickness comprised between 1 mm and 3 mm, in particular of 2 mm.

6. End-effector (1) according to any one of claims 1 to 5, wherein the stem element (2) has a main body having a width comprised between 5 mm and 10 mm, in particular 9 mm in order to be compatible with standard laboratory glassware for medium and high throughput screening.

7. End-effector (1) according to any one of claims 1 to 6, wherein
a. the stem element (2) comprises a radial flap (9) at the second end (7); and/or
b. the deformable membrane (5) comprises an extruded confining rim (10).

8. End-effector (1) according to any one of claims 1 to 7, wherein in the deflated configuration the surface of the deformable membrane (5) is flat and in the inflated configuration the surface of the deformable membrane is curved and is protruding outwards.

9. System (11) for cell volumetric stimulation comprising:
a plurality of end-effectors (1) according to any one of claims 1 to 8; and
at least a top plate (12) configured to position each of the plurality of end-effectors (1) in a corresponding well structure (13) of a multi-well plate.

10. System according to claim 9, wherein the top plate (12) is configured as a rigid lid, in particular of plastic, for ensuring both protection from contamination and recirculation of metabolic gasses and oxygen in a growth medium (16) present in the well structure (13).

11. Apparatus (15) for cell volumetric stimulation comprising:
at least an end-effector (1) according to any one of claims 1 to 8 or at least a system (11) according to any one of claims 9 to 10; and
an actuation system (14) having an air transmission system (4) housed at least in part in the stem element (2) of the end-effector (1) for generating volumetrically constant, monotonous, or user defined rhythmic stimulation on cells, wherein in particular the actuation system (14) comprises mechanical, electronic and/or hydraulic components (28, 29, 30) for measuring and controlling physical parameters on the deformable membrane (5) and a microcontroller (27) connected to said mechanical, electronic and/or hydraulic components (28, 29, 30).

12. Apparatus (15) according to claim 11, further comprising an analogical or digital I/O interface for controlling and setting deformation parameters of the cells.

13. Method for manufacturing an end-effector (1) for cell volumetric stimulation according to any one of claims 1 to 8, the method comprising:
- forming (S101) a membrane layer (21) and a stem layer (22) by sequential pouring bi-component silicone rubber in a membrane mold (18) and a stem mold (19), respectively, and
- interrupting (S102) the sequential pouring by the insertion of a tin sphere (20) to form an internal cavity between the membrane layer (21) and the stem layer (22), the internal cavity functioning as the air chamber (6).

14. Method according to claim 13, comprising:
- coating (S201) a membrane mold (18) with resin, in particular polyepoxides, and curing for a first curing time, in particular 72 hours;
- coating (S202) the membrane mold (18) with released release agents (24);
- pouring (S203) a first quantity of mixed bi-component silicone rubber in the membrane mold (18) and curing for a second curing time, in particular 3 hours, to form the membrane layer (21);
- placing (S204) a guide (25) for the tin sphere, pouring a second quantity of mixed bi-component silicone rubber and curing for a third curing time, in particular 3 hours;
- removing (S205) the guide (25) and inserting the tin sphere (20);
- pouring (S206) a third quantity of mixed bi-component silicone rubber and curing for a fourth curing time, in particular 3 hours;
- placing (S207) a pinned plane (26) in the stem mold (19) to generate a needle guide; and
- pouring (S208) a silicone rubber harder than the mixed bi-component silicone rubber in the stem mold (19) and curing for a fifth curing time, in particular 12 hours, to form the stem layer (22).

15. Method for cell volumetric stimulation using the end-effector (1) according to any one of claims 1 to 8, or the system (11) according to any one of claims 9 to 10, or the apparatus (15) according to any one of claims 11 to 12, the method comprising:
- providing (S301) at least one end-effector (1);
- seeding the cells (17) (S302) on an external surface of the deformable membrane (5) of said end-effector (1);
- placing (S303) the end-effector (1) at least partially in a growth medium (16) so that at least the cells (17) are completely immersed in said growth medium (16); and
- inserting (S304) air into the air chamber (6) and expelling air from the air chamber (6) in a continuous cycle, thereby providing a pressure and volume variation of the deformable membrane (5) and obtaining cells stimulation.
